# EUROPEAN PATENT SPECIFICATION

(11) **EP 3 922 200 B1**
(45) Date of publication and mention of the grant of the patent: **26.04.2023**
(21) Application number: 21178427.7
(22) Date of filing: 09.06.2021
(51) Int. Cl.: A61B 18/14, A61B 18/18, A61M 35/00, A61N 1/40, A61N 7/00, A61N 5/02, A61B 18/00, A61M 37/00

(54) **APPARATUS FOR REGENERATIVE SKIN TREATMENT AND LIPOLYSIS**
VORRICHTUNG ZUR REGENERATIVEN HAUTBEHANDLUNG UND LIPOLYSE
APPAREIL DE TRAITEMENT RÉGÉNÉRATIF DE LA PEAU ET DE LIPOLYSE

(30) Priority: 11.06.2020 IT 202000013984
(43) Date of publication of application: 15.12.2021
(73) Proprietor: BIOTEC ITALIA S.R.L., 36031 Dueville (VI) (IT)
(72) Inventor: FERRARI, Fulvio, 35137 Padova (PADOVA) (IT)
(74) Representative: Forattini, Amelia

(56) References cited:
- WO-A1-2016/168435
- KR-A- 20190 011 797
- US-A1- 2007 055 179
- US-A1- 2011 077 451
- US-A1- 2019 134 414
- US-A1- 2019 255 347
- US-B1- 6 500 141

## Description

The present invention relates to an apparatus for regenerative skin treatment and lipolysis.

As is known, the most common treatment for fighting skin aging, cellulite and fat has been for many years a surgical procedure in its many forms.

However, with the increase in the demand for cosmetic rejuvenation and a strong desire of the patients to achieve aesthetic improvement with minimal risk and rapid recovery, there has been a strong growth in non-ablative procedures, which have the purpose of rejuvenating the skin and the subcutaneous tissue, in addition to reducing the adipose layer, safely and effectively.

Although various non-ablative systems have been approved by the Food and Drug Administration (FDA), in the United States of America clinical results have been generally scarcely satisfactory, with most subjects showing only a slight improvement.

Non-ablative procedures have also demonstrated evident limitations in some safety aspects.

For example, in the case of energies used to treat surface and deep tissue volume, it has been found that it is particularly difficult to cool the surface tissue portion in a manner that is efficient and functional to the therapeutic effect.

US2019134414A1 discloses a method of soft tissue treatment of a patient by placing an applicator onto a surface of a soft tissue; the applicator has an RF electrode and a dielectric material having a vacuum cup and a dielectric material under the RF electrode; the dielectric material under the electrode has an absolute value of difference between polarization factor below center of the RF electrode and below edges of the RF electrode in a range from to 0.10005 mm to 19 800 mm; the soft tissue is heated via the RF electrode; vacuum is applied into a cavity under the applicator with changing pressure value inside the cavity under the applicator compared to pressure in the room during the treatment in range from 0.01 kPa to 100 kPa.

WO2016168435A1 discloses a radio frequency system for selectively heating skin and subcutaneous fat tissues using a single frequency dual mode antenna device; the antenna device generates electric fields that are either mostly tangent, or mostly normal, to the skin surface, resulting in either predominantly heating skin or predominantly heating fat.

US6500141B1 discloses an apparatus for treating superficial soft tissue with ultrasound; the apparatus has an ultrasonic generation unit and an applicator, by means of which the ultrasound can be irradiated from an applicator surface facing the body surface from outside through the body surface into the body tissue; a suction apparatus for sucking in the body surface against the applicator surface is provided.

KR20190011797A discloses an RF energy transfer device having: an RF generator, an applicator transferring the RF energy to a target volume located in the body, and a controller for differentially controlling the magnitude of RF energy delivered to each temperature-rising section so as to reach a treatment temperature by dividing a temperature range into a plurality of temperature rising sections during RF energy transfer to the target volume; the RF energy transfer device should reduce the total time taken while maintaining the therapeutic effect of adipocytes.

The aim of the present invention is to provide an apparatus for regenerative skin treatment and lipolysis that overcome the drawbacks of the cited prior art.

Within the scope of this aim, a particular object of the invention is to provide an apparatus capable of utilizing the synergy of technologies that are non-ablative and have an acknowledged effectiveness in order to be able to treat the various volumes of tissue in order to ensure a greater therapeutic effect than the systems used so far for rejuvenation and redefinition of adipose tissue.

A further object of the invention is to provide an apparatus capable of facilitating the treatments and of performing broad-range non-ablative procedures in order to obtain tangible results in relatively short times.

A further object of the invention is to provide an apparatus which, by virtue of their particular constructive characteristics, are capable of giving the greatest assurances of reliability and safety in use.

This aim and these objects, as well as others which will become better apparent hereinafter, are achieved by an apparatus as claimed in the appended claims.

Further characteristics and advantages will become better apparent from the description of preferred but not exclusive embodiments of an apparatus for regenerative skin treatment and lipolysis according to the invention, illustrated by way of non-limiting example in the accompanying drawings, wherein:

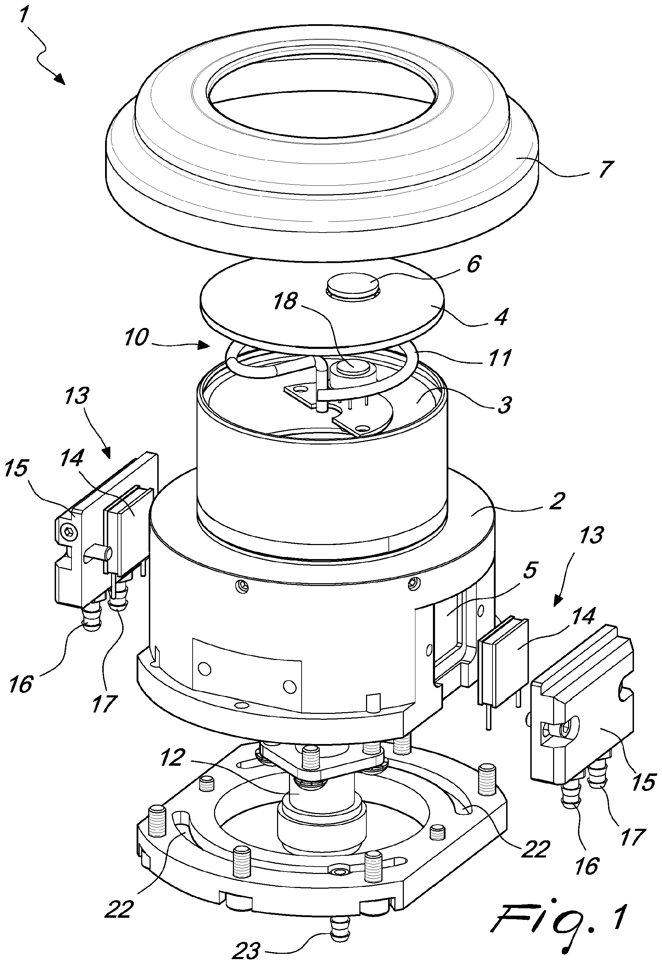

Figure 1 is an exploded view of a device which is part of an apparatus according to the invention;

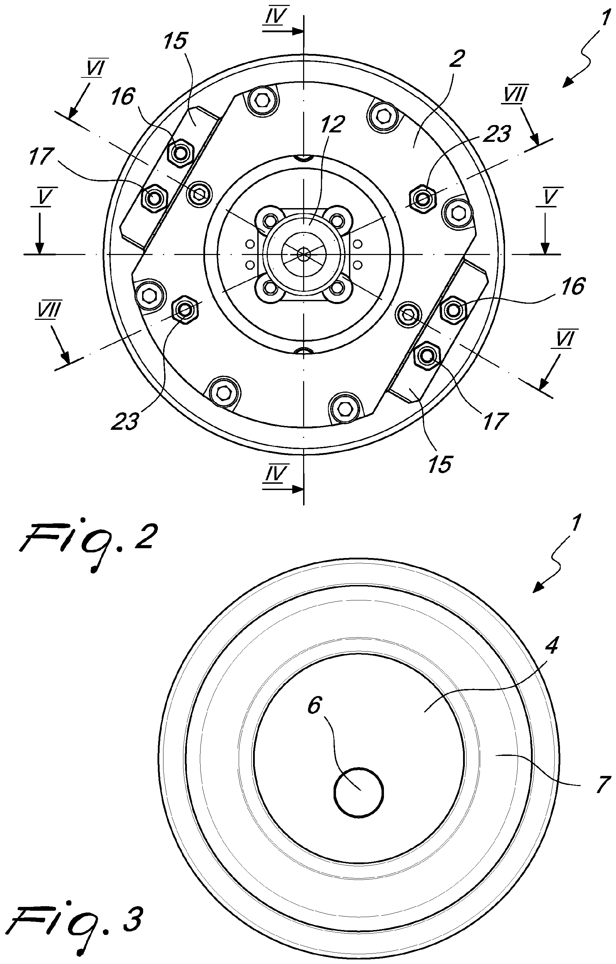

Figure 2 is a top view of the device;
Figure 3 is a bottom view of the device;

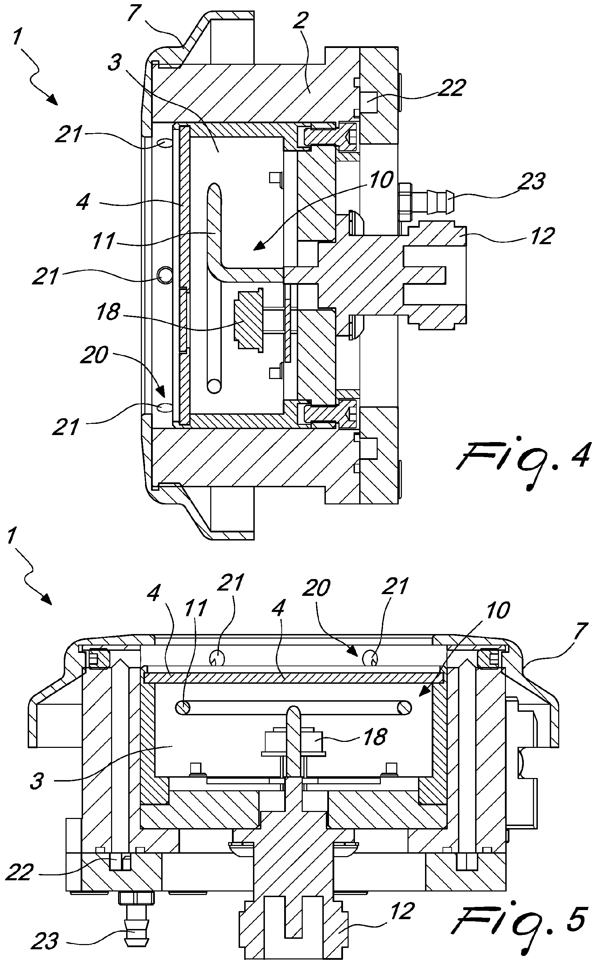

Figure 4 is a sectional view, taken along the plane IV-IV of Figure 2;
Figure 5 is a sectional view, taken along the plane V-V of Figure 2;

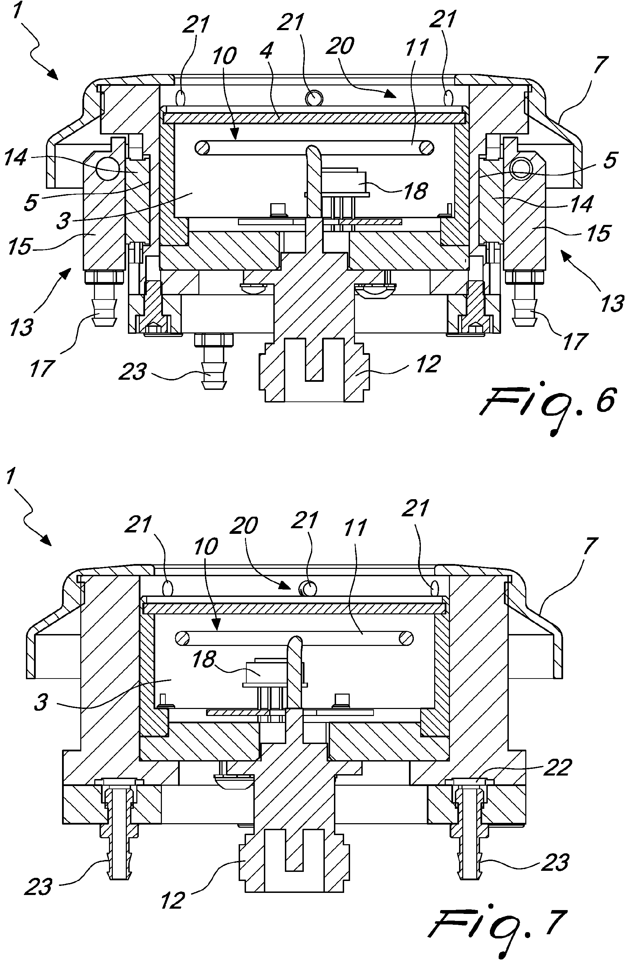

Figure 6 is a sectional view, taken along the plane VI-VI of Figure 2;
Figure 7 is a sectional view, taken along the plane VII-VII of Figure 2;
Figure 8 is a block diagram of an apparatus according to the invention.

With reference to the cited figures, a device for regenerative skin treatment and lipolysis is designated generally by the reference numeral 1.

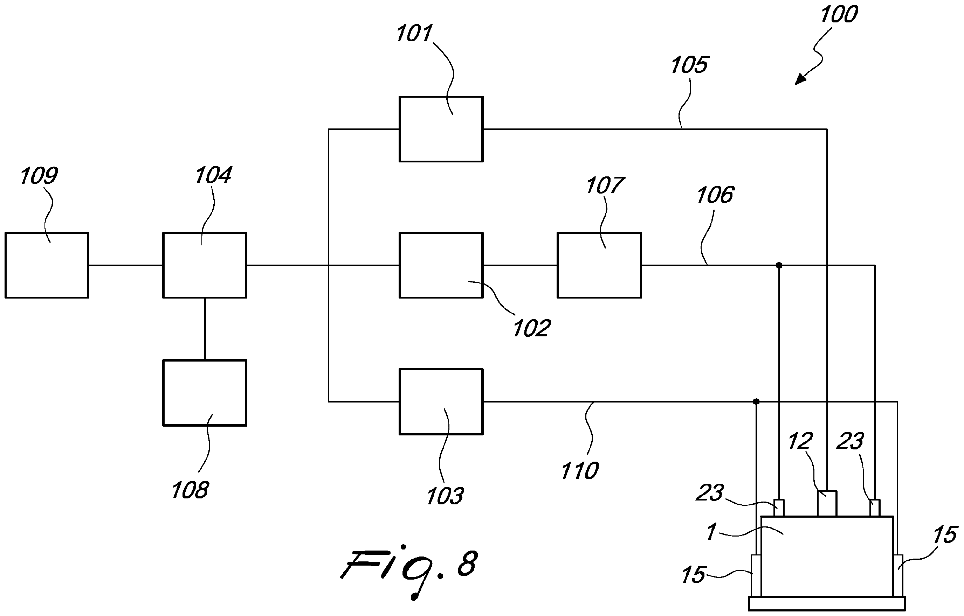

The device 1 can be used in combination with an apparatus 100, shown schematically in Figure 8, and described hereinafter, according to the present invention.

The device 1 includes a substantially cylindrical and substantially hollow body 2 designed to be arranged in contact with a skin area of a patient to be treated.

In this regard, the contact surface of the body 2 is preferably covered by a ring 7 which can be removed and sanitized in order to give assurance to the patient of high hygiene conditions or to be replaced in case of damage.

The body 2 is preferably inserted in a handholdable support, not shown in the drawings, so that it can be easily handled and directed by an operator.

The body 2 has a substantially cylindrical chamber 3. The substantially cylindrical chamber 3 is delimited by a removable wall 4; the end of the removable wall is designed to rest against the skin area to be treated.

The device 1 includes a vibrational energy emission means 10, capable of selectively irradiating vibrational energy to a deep layer of the skin indicatively corresponding to the hypodermis and to the deepest part of the dermis of the skin area to be treated.

The device 1 also includes a treatment fluid emission means 20, capable of selectively spraying treatment fluid to a surface layer of the skin indicatively corresponding to the epidermis and to the less deep part of the dermis of the skin area to be treated.

The expression "selectively irradiating" means that the vibrational energy emission means 10 is configured to irradiate the skin area to be treated with this energy, by generating the energy or transmitting the energy, in at least one first operating condition and to stop or prevent this irradiation in at least one second operating condition. The vibrational energy emission means is also selectively configurable at least in the first and second operating conditions.

Likewise, the expression "selectively spraying" means that the treatment fluid emission means 20 is configured to spray fluid on the skin area to be treated, by generating the fluid or conveying the fluid, in at least one first operating condition and to stop or prevent this spraying in at least one second operating condition. The treatment fluid emission means is selectively configurable at least in the first and second operating conditions.

The vibrational energy emission means 10 substantially comprises a wave emitter 11 which is electrically connected to a vibrational energy generation means 101 with which the apparatus 100 is equipped. The wave emitter 11 is electrically detachably connected to the vibrational energy generation means 101 by means of an electrical connector 12 and a cable 105.

The wave emitter 11 is arranged inside the chamber 3 and faces the removable wall 4, which is preferably made of a material that is substantially transparent to the waves emitted by the vibrational energy emission means 10.

The wave emitter 11 shown in the drawings is an antenna adapted to emit microwaves or radiofrequency; alternatively, the wave emitter 111 is for example an ultrasound generator.

In fact, depending on the treatments and the desired effects on the patient, the waves emitted by the vibrational energy emission means 10 are chosen from the group that includes electromagnetic waves, acoustic waves and combinations thereof.

In greater detail, the electromagnetic waves generated by the vibrational energy generation means 101 have a frequency comprised between about 300 kHz and about 3 GHz.

For some uses, however, the operating band is narrower and the electromagnetic waves have a frequency preferably comprised between about 2.4 GHz and about 2.5 GHz, and even more preferably centered on 2.45 GHz.

The acoustic waves generated by the vibrational energy generation means 101 instead have a frequency higher than about 20 kHz.

Preferably, the chamber 3 is cooled by means of a chamber cooling means 13 which allows to dissipate the excess heat produced by the vibrational energy emission means 10.

This allows both to cool the skin area to be treated, in a manner that is efficient and functional to the therapeutic effect, and to avoid burns or inconvenience to the patient.

Specifically, the chamber cooling means 13 includes one or more, preferably two, thermoelectric devices 14, for example Peltier cells, which are arranged outside the body 2 in adapted receptacles 5.

Each thermoelectric device 14 is associated with a cooling system 15 supplied with cooling fluid, typically water and/or glycol, which circulates between an input connector 16 and an output connector 17.

In order to increase the circulation rate of the cooling fluid, and therefore the dissipating power of the chamber cooling means 13, each cooling system 15 is fluidically connected to a recirculation means 103 with which the apparatus 100 is equipped. Each cooling system 15 is detachably connected to the recirculation means 103, by means of tubes 110.

The temperature of the chamber 3, and therefore also of the skin area to be treated, is controlled by means of a temperature sensor 18 which is arranged inside the chamber 3 and faces a window 6 formed in the removable wall 4.

The temperature sensor 18 is electrically connected to a control system 104 with which the apparatus 100 is equipped and which has the task of controlling the vibrational energy emission means 10 and the treatment fluid emission means 20, for example by bringing them selectively into the first and/or second operating conditions described above.

The temperature sensor 18 is detachably connected to the control system 104 by means of the electrical connector 12.

The treatment fluid emission means 20 is made of a plurality of diffusion holes 21 fluidically connected to a treatment fluid supply means 102 with which the apparatus 100 is equipped.

The diffusion holes 21 are arranged substantially at the edge of the body 2 that is proximal to the removable wall 4, outside the chamber 3, so as to generate a flow of treatment fluid which skims the skin area to be treated.

The diffusion holes 21 are fluidically connected to each other by means of a diffuser channel 22, which is preferably formed within the thickness of the body 2.

The diffuser channel 22 is provided with one or more, two in the specific case, input connectors 23 for supplying the treatment fluid by means of the treatment fluid supply means 102; the input connectors 23 are fluidically connected to the treatment fluid supply means 102 by ducts 106.

Depending on the treatments and on the desired effects on the patient, the treatment fluid emitted by the treatment fluid emission means 20 is chosen from the group that includes ozone, enriched oxygen, atomized liquid, active ingredients, and combinations thereof.

Preferably, the treatment fluid is cooled by a treatment fluid cooling means 107 equipped to the apparatus 100.

This allows to aid the action of the chamber cooling means 13 and to cool the skin area to be treated both in a manner that is efficient and functional for the therapeutic effect and in order to avoid burns or inconvenience to the patient.

Preferably, the temperature of the treatment fluid, and therefore also of the skin area to be treated, is controlled by virtue of the temperature sensor 18 and the control system 104.

According to the invention, an apparatus 100 for regenerative skin treatment and lipolysis is used in combination with the device 1.

The apparatus 100 substantially includes the vibrational energy generation means 101, the treatment fluid supply means 102, and the corresponding control system 104, to which the device 1 is connected.

In greater detail, the device 1 is electrically connected to the vibrational energy generation means 101, and is fluidically connected to the treatment fluid supply means 102.

The device 1 is electrically detachably connected to the vibrational energy generation means 101 by means of the electrical connector 12 and of the cable 105.

The device 1 is detachably fluidically connected to the treatment fluid supply means 102 by means of the input connectors 23 and the ducts 106.

Preferably, the treatment fluid supply means 102 is constituted by a device capable of producing enriched oxygen starting from the surrounding environment or from an ozone generator.

The apparatus 100 also includes the recirculation means 103 and the treatment fluid cooling means 107.

The apparatus 100 is powered by an electric power supply means 108.

The operator can interact with the apparatus 100, and therefore also with the device 1, by virtue of an input/output interface means 109 connected to the control system 104.

In the specific case, the input/output interface means 109 includes, for example, a touchscreen or any other interaction system.

The operation of the device is described hereinafter.

After adequately preparing the patient, the device 1 is rested on the skin area to be treated and the operator can start the treatment.

The vibrational energy generation means 10 thus begins to irradiate the deep layer of the skin that belongs to the skin area to be treated, with the waves that originate from the wave emitter 11.

Simultaneously, the treatment fluid emission means 20 begins to spray the surface layer of the skin that belongs to the skin area to be treated, with the treatment fluid.

The deep tissue volume of the skin area to be treated is then irradiated with vibrational energy in the form of electromagnetic waves or acoustic waves.

In the case of electromagnetic waves with a frequency that can vary between about 300 kHz and about 3 GHz, the energy can be applied directly to the tissues, so that it can be absorbed or even retransmitted for a variety of purposes.

This allows to selectively heat the deep dermis and the subcutaneous fibrous septa, causing the breakup of the hydrogen bonds in the collagen fibers and the subsequent denaturation of the collagen.

The subsequent inflammatory and proliferative healing steps thus stimulate the new deposition and restructuring of the collagen fibers, with consequent skin rejuvenation.

The use of electromagnetic waves in the frequency range comprised between about 2.4 GHz and about 2.5 GHz, and in particular around 2.45 GHz, allows to interact effectively with the biological molecules of the adipose tissue and to generate localized and controlled heat, which is absorbed by selected biological targets, such as water and fat, by means of a biophysical process known as "dielectric heating".

Moreover, the electromagnetic waves induce a specific activation of numerous transport mechanisms within the cell membranes of adipocytes.

The main consequence of localized dielectric heating is the immediate decantation of the cytoplasm of adipocytes and irreversible damage to the cell membrane.

The electromagnetic waves act directly on the subdermal adipose tissue, promoting the heating of the adipocyte cells without affecting the less deep dermal-epidermal layers.

As a consequence, the action of macrophages is activated, with reduction of the subdermal adipose tissue and reduction of the adipose panniculus.

Advantageously, with the vibrational energy emission means 10 it is possible to supply thermal energy to tissues in a nonspecific manner, differently from lasers, which depend on chromophores.

Ultrasounds can instead be used for their ablative action (HIFU) or to produce heat in a controlled form and to convey active ingredients in the skin with sonoporation methods.

In the treatment of adiposities, in particular, ultrasound utilizes the phenomenon of cavitation.

In practice, ultrasounds at clearly defined frequencies are generated which reach the subcutaneous adipose tissue, causing pressure and temperature variations in the interstitial fluid that is present between the adipocytes.

These variations cause the rapid forming of vapor microbubbles.

These bubbles, under the influence of the ultrasounds, increase and decrease their volume very rapidly, to the point of imploding.

The implosions of the microbubbles generate very high and localized mechanical energy and pressures, which cause the rupture of the cell membranes of the surrounding adipocytes, with consequent release of the fats contained therein.

The lipids that have thus been released from the destroyed adipocytes are removed from the treated area by means of the lymphatic system, to be then processed by the liver and subsequently eliminated, exactly as occurs for fats intaken during eating.

The volume of surface tissue of the skin area to be treated is instead sprayed with cold treatment fluid in the form of a solution of gas, for example oxygen, and a dispersion of the gas in a liquid, for example water.

The infusion of oxygen and liquid allows the flows of pure oxygen (up to 96% purity) to reach the surface layer of the skin and to be absorbed by the dermis and epidermis.

Oxygen has various beneficial effects on the skin: it is capable of improving cell metabolism, of accelerating healing processes, of reducing skin irritations and of producing an anti-inflammatory effect.

Oxygen is also capable of producing a neoangiogenic effect, i.e., it promotes the release of the vascular endothelial growth factor (VEGF) and of other factors involved in neoangiogenesis.

Oxygen also has an antibacterial effect by virtue of its ability to release reactive oxygen species (ROS), which are extremely toxic compounds since they degrade cell membranes and cause the death of pathogenic microorganisms.

The apparatus 100 according to the invention allow to utilize the application synergy of non-ablative technologies of acknowledged effectiveness such as irradiation with electromagnetic waves or acoustic waves and oxygen therapy.

The apparatus 100 according to the invention allow to treat the various volumes of tissue in order to ensure a greater therapeutic effect than the systems used currently for rejuvenation and redefinition of adipose tissue, ensuring safety, by means of the cooling of the volume of surface tissue monitored by a temperature sensor, but at the same time using the therapeutic action of the medium used for cooling.

The apparatus according to the invention allow to facilitate the treatments and to perform broad-range non-ablative procedures, obtaining tangible results in relatively short times.

The apparatus according to the invention, also by virtue of the system for the control of the temperature of the skin area being treated, are capable of giving the greatest assurances of reliability and safety in use.

The invention therefore achieves the intended aim and objects.

## Claims

1. An apparatus for regenerative skin treatment and lipolysis comprising a vibrational energy generation means (10), a treatment fluid supply means (102) and a control system (104) for the control of said vibrational energy generation means (10) and of said treatment fluid supply means (102); at least one device (1) for regenerative skin treatment and lipolysis comprising a body (2) adapted to be placed in contact with a skin area to be treated, said device (1) comprising a vibrational energy emission means adapted to selectively irradiate a deep layer of said skin area to be treated, and a treatment fluid emission means (20) emitting a treatment fluid; said treatment fluid emission means (20) being adapted to selectively spray treatment fluid to a surface layer of said skin area to be treated; said apparatus being **characterized in that** said treatment fluid supply means (102) comprises at least one device capable of producing enriched oxygen starting from the surrounding environment or an ozone generator.

2. The apparatus according to claim 1, **characterized in that** it comprises an electric power supply means (108) and an input/output interface means (109) in order to allow the interaction of an operator with said system for the control of said vibrational energy generation means (10) and of said treatment fluid supply means (102), and the interaction of said operator with said device.

3. The apparatus according to one or more of the preceding claims, **characterized in that** said body (2) of said device (1) comprises a chamber (3) containing said vibrational energy emission means (10); said chamber (3) being at least partially delimited by a removable wall (4) which is mechanically associated with said body (2); said removable wall (4) being substantially transparent to said vibrational energy.

4. The apparatus according to one or more of the preceding claims, **characterized in that** said vibrational energy emission means (10) comprises at least one wave emitter (11) adapted to be electrically connected to a vibrational energy generating means (10) generating said vibrational energy, said wave emitter (11) being arranged so as to face said removable wall (4), inside said chamber (3).

5. The apparatus according to one or more of the preceding claims, **characterized in that** said waves are chosen from the group comprising electromagnetic waves, acoustic waves, and combinations thereof.

6. The apparatus according to one or more of the preceding claims, **characterized in that** said electromagnetic waves have a frequency comprised between about 300 kHz and about 3 GHz.

7. The apparatus according to one or more of the preceding claims, **characterized in that** said acoustic waves have a frequency higher than about 20 kHz.

8. The apparatus according to one or more of the preceding claims, **characterized in that** said treatment fluid emission means (20) comprises a plurality of diffusion holes (21) arranged in fluidic connection with said treatment fluid supply means (102), said diffusion holes (21) being arranged substantially at the edge of said body (2) that is proximal to said removable wall (4), outside said chamber (3), so as to generate a flow of said treatment fluid toward said skin area to be treated.

9. The apparatus according to one or more of the preceding claims, **characterized in that** said treatment fluid emission means (20) comprises at least one diffuser channel (22) in fluidic connection with said diffusion holes (21), said diffuser channel (22) being formed within the thickness of said body (2) and having one or more input connectors (23) for supplying said treatment fluid by virtue of said treatment fluid supply means (102).

10. The apparatus according to one or more of the preceding claims, **characterized in that** said treatment fluid is chosen from the group comprising ozone, enriched oxygen, atomized liquid, active ingredients and combinations thereof.

11. The apparatus according to one or more of the preceding claims, **characterized in that** said treatment fluid is cooled by a treatment fluid cooling means (107).

12. The apparatus according to one or more of the preceding claims, **characterized in that** it comprises a chamber cooling means (13).

13. The apparatus according to one or more of the preceding claims, **characterized in that** said chamber cooling means (13) comprises one or more thermoelectric devices (14) arranged outside said body (2), each of said thermoelectric devices (14) being associated with a cooling system (15) supplied with a cooling fluid which circulates between an input connector (16) and an output connector (17).

14. The apparatus according to one or more of the preceding claims, **characterized in that** it comprises at least one temperature sensor (18) adapted to measure the temperature of said skin area to be treated and to transmit the data to said control system (104) for the control of said vibrational energy generation means (10) and of said treatment fluid supply means (102), said temperature sensor (18) being arranged so as to face a window (6) provided in said removable wall (4), inside said chamber (3).

## Patentansprüche

1. Eine Vorrichtung zur regenerativen Hautbehandlung und Lipolyse, die Folgendes umfasst: ein Schwingungsenergie-Erzeugungsmittel (10), ein Behandlungsfluid-Zuführmittel (102) und ein Steuerungssystem (104) zur Steuerung des Schwingungsenergie-Erzeugungsmittels (10) und des Behandlungsfluid-Zuführmittels (102); wobei mindestens ein Gerät (1) zur regenerativen Hauptbehandlung und Lipolyse einen Körper (2) umfasst, der ausgebildet ist, um in Kontakt mit einem zu behandelnden Hautbereich positioniert zu werden; wobei das Gerät (1) ein Schwingungsenergie-Abgabemittel umfasst, das ausgebildet ist, um wahlweise eine tiefe Schicht des zu behandelnden Hautbereichs zu bestrahlen, und ein Behandlungsfluid-Abgabemittel (20), das ein Behandlungsfluid abgibt; wobei das Behandlungsfluid-Abgabemittel (20) ausgebildet ist, um wahlweise Behandlungsfluid auf eine Oberflächenschicht des zu behandelnden Hautbereichs aufzusprühen; wobei die Vorrichtung **dadurch gekennzeichnet ist, dass** das Behandlungsfluid-Zuführmittel (102) mindestens ein Gerät umfasst, das in der Lage ist, ausgehend von der Umgebung oder einem Ozongenerator angereicherten Sauerstoff zu erzeugen.

2. Die Vorrichtung gemäß Anspruch 1, **dadurch gekennzeichnet, dass** sie ein Mittel (108) zur elektrischen Stromversorgung und ein Eingabe-/Ausgabe-Schnittstellenmittel (109) umfasst, um die Interaktion eines Bedieners mit dem System zur Steuerung des Schwingungsenergie-Erzeugungsmittels (10) und des Behandlungsfluid-Zuführmittels (102) sowie die Interaktion des Bedieners mit dem Gerät zu ermöglichen.

3. Die Vorrichtung gemäß einem oder mehreren der obigen Ansprüche, **dadurch gekennzeichnet, dass** der Körper (2) des Geräts (1) einen Raum (3) umfasst, welcher das Schwingungsenergie-Erzeugungsmittel (10) enthält; wobei der Raum (3) zumindest teilweise durch eine abnehmbare Wand (4) begrenzt ist, die mechanisch mit dem Körper (2) verbunden ist; wobei die abnehmbare Wand (4) zumindest teilweise durchlässig für die Schwingungsenergie ist.

4. Die Vorrichtung gemäß einem oder mehreren der obigen Ansprüche, **dadurch gekennzeichnet, dass** das Schwingungsenergie-Erzeugungsmittel (10) mindestens einen Wellenstrahler (11) umfasst, der ausgebildet ist, um elektrisch mit einem Schwingungsenergie-Erzeugungsmittel (10) verbunden zu werden, das die Schwingungsenergie erzeugt; wobei der Wellenstrahler (11) innerhalb des Raumes (3), der abnehmbaren Wand (4) zugewandt, angeordnet ist.

5. Die Vorrichtung gemäß einem oder mehreren der obigen Ansprüche, **dadurch gekennzeichnet, dass** die Wellen gewählt sind aus der Gruppe, die elektromagnetische Wellen, akustische Wellen und Kombinationen davon umfasst.

6. Die Vorrichtung gemäß einem oder mehreren der obigen Ansprüche, **dadurch gekennzeichnet, dass** die elektromagnetischen Wellen eine Frequenz zwischen ungefähr 300 kHz und ungefähr 3 GHz haben.

7. Die Vorrichtung gemäß einem oder mehreren der obigen Ansprüche, **dadurch gekennzeichnet, dass** die akustischen Wellen eine Frequenz haben, die höher als ungefähr 20 kHz ist.

8. Die Vorrichtung gemäß einem oder mehreren der obigen Ansprüche, **dadurch gekennzeichnet, dass** das Behandlungsfluid-Abgabemittel (20) eine Vielzahl von Diffusionsöffnungen (21) umfasst, angeordnet in Fluidverbindung mit dem Behandlungsfluid-Zuführmittel (102), wobei die Diffusionsöffnungen (21) im Wesentlichen am Rand des Körpers (2) angeordnet sind, der an die abnehmbare Wand (4) angrenzt, außerhalb des Raumes (3), um einen Strom des Behandlungsfluids zu dem zu behandelnden Hautbereich zu erzeugen.

9. Die Vorrichtung gemäß einem oder mehreren der obigen Ansprüche, **dadurch gekennzeichnet, dass** das Behandlungsfluid-Abgabemittel (20) mindestens einen Diffusorkanal (22) in Fluidverbindung mit den Diffusionsöffnungen (21) umfasst, wobei der Diffusorkanal (22) in der Dicke des Körpers (2) geformt ist und ein oder mehrere Eingangsverbindungsstücke (23) für die Zufuhr des Behandlungsfluids durch die Behandlungsfluid-Zuführmittel (102) hat.

10. Die Vorrichtung gemäß einem oder mehreren der obigen Ansprüche, **dadurch gekennzeichnet, dass** das Behandlungsfluid gewählt ist aus der Gruppe, die Ozon, angereicherten Sauerstoff, zerstäubte Flüssigkeit, Wirkstoffe und Kombinationen davon umfasst.

11. Die Vorrichtung gemäß einem oder mehreren der obigen Ansprüche, **dadurch gekennzeichnet, dass** das Behandlungsfluid durch eine Behandlungsfluid-Kühlvorrichtung (107) gekühlt wird.

12. Die Vorrichtung gemäß einem oder mehreren der obigen Ansprüche, **dadurch gekennzeichnet, dass** sie eine Raum-Kühlvorrichtung (13) umfasst.

13. Die Vorrichtung gemäß einem oder mehreren der obigen Ansprüche, **dadurch gekennzeichnet, dass** die Raum-Kühlvorrichtung (13) ein oder mehrere thermoelektrische Geräte (14) umfasst, die außerhalb des Körpers (2) angeordnet sind, wobei jedes der thermoelektrischen Geräte (14) mit einem Kühlsystem (15) verbunden ist, das mit einem Kühlfluid versorgt wird, welches zwischen einer Eingangs-Anschlussvorrichtung (16) und einer Ausgangs-Anschlussvorrichtung (17) zirkuliert.

14. Die Vorrichtung gemäß einem oder mehreren der obigen Ansprüche, **dadurch gekennzeichnet, dass** sie mindestens einen Temperatursensor (18) umfasst, der ausgebildet ist, um die Temperatur des zu behandelnden Hautbereichs zu messen und die Daten an das Steuerungssystem (104) für die Steuerung des Schwingungsenergie-Erzeugungsmittels (10) und des Behandlungsfluid-Zuführmittels (102) zu senden, wobei der Temperatursensor (18) einem in der abnehmbaren Wand (4) angebrachten Fenster (6) zugewandt, innerhalb des Raumes (3), angeordnet ist.

## Revendications

1. Appareil de traitement régénératif de la peau et de lipolyse comprenant un moyen de génération d'énergie vibratoire (10), un moyen d'alimentation en fluide de traitement (102) et un système de commande (104) pour la commande dudit moyen de génération d'énergie vibratoire (10) et dudit moyen d'alimentation en fluide de traitement (102); au moins un dispositif (1) de traitement régénératif de la peau et de lipolyse comprenant un corps (2) apte à être placé en contact avec une zone de la peau à traiter, ledit dispositif (1) comprenant un moyen d'émission d'énergie vibratoire apte à irradier sélectivement une couche profonde de ladite zone de peau à traiter, et un moyen d'émission de fluide de traitement (20) émettant un fluide de traitement ; ledit moyen d'émission de fluide de traitement (20) étant apte à pulvériser sélectivement ledit fluide de traitement sur une couche superficielle de ladite zone de peau à traiter ; ledit appareil étant **caractérisé en ce que** ledit moyen d'alimentation en fluide de traitement (102) comprend au moins un dispositif apte à produire de l'oxygène enrichi à partir de l'environnement ambiant ou d'un générateur d'ozone.

2. Appareil selon la revendication 1, **caractérisé en ce qu'**il comprend un moyen d'alimentation en courant électrique (108) et un moyen d'interface d'entrée/sortie (109) afin de permettre l'interaction d'un opérateur avec ledit système pour la commande dudit moyen de génération d'énergie vibratoire (10) et dudit moyen d'alimentation en fluide de traitement (102), et l'interaction dudit opérateur avec ledit dispositif.

3. Appareil selon une ou plusieurs des revendications précédentes, **caractérisé en ce que** ledit corps (2) dudit dispositif (1) comprend une chambre (3) contenant ledit moyen d'émission d'énergie vibratoire (10) ; ladite chambre (3) étant au moins partiellement délimitée par une paroi amovible (4) qui est associée mécaniquement audit corps (2); ladite paroi amovible (4) étant substantiellement transparente à ladite énergie vibratoire.

4. Appareil selon une ou plusieurs des revendications précédentes, **caractérisé en ce que** ledit moyen d'émission d'énergie vibratoire (10) comprend au moins un émetteur d'ondes (11) apte à être connecté électriquement à un moyen de génération d'énergie vibratoire (10) générant ladite énergie vibratoire, ledit émetteur d'ondes (11) étant disposé de manière à être en face de ladite paroi amovible (4) à l'intérieur de ladite chambre (3).

5. Appareil selon une ou plusieurs des revendications précédentes, **caractérisé en ce que** lesdites ondes sont choisies dans le groupe comprenant les ondes électromagnétiques, les ondes acoustiques, et leurs combinaisons.

6. Appareil selon une ou plusieurs des revendications précédentes, **caractérisé en ce que** lesdites ondes électromagnétiques ont une fréquence comprise entre environ 300 kHz et environ 3 GHz.

7. Appareil selon une ou plusieurs des revendications précédentes, **caractérisé en ce que** lesdites ondes acoustiques ont une fréquence supérieure à environ 20 kHz.

8. Appareil selon une ou plusieurs des revendications précédentes, **caractérisé en ce que** ledit moyen d'émission de fluide de traitement (20) comprend une pluralité de trous de diffusion (21) disposés en connexion fluidique avec ledit moyen d'alimentation en fluide de traitement (102), lesdits trous de diffusion (21) étant disposés substantiellement au bord dudit corps (2) qui est proximal à ladite paroi amovible (4) à l'extérieur de ladite chambre (3) afin de générer un flux dudit fluide de traitement vers ladite zone de peau à traiter.

9. Appareil selon une ou plusieurs des revendications précédentes, **caractérisé en ce que** ledit moyen d'émission de fluide de traitement (20) comprend au moins un canal de diffusion (22) en connexion fluidique avec lesdits trous de diffusion (21), ledit canal de diffusion (22) étant formé dans l'épaisseur dudit corps (2) et comportant une ou plusieurs connexions d'entrée (23) pour fournir ledit fluide de traitement par l'intermédiaire dudit moyen d'alimentation en fluide de traitement (102).

10. Appareil selon une ou plusieurs des revendications précédentes, **caractérisé en ce que** ledit fluide de traitement est choisi dans le groupe comprenant l'ozone, l'oxygène enrichi, un liquide atomisé, des ingrédients actifs et leurs combinaisons.

11. Appareil selon une ou plusieurs des revendications précédentes, **caractérisé en ce que** ledit fluide de traitement est refroidi par un moyen de refroidissement de fluide de traitement (107).

12. Appareil selon une ou plusieurs des revendications précédentes, **caractérisé en ce qu'**il comprend un moyen de refroidissement de chambre (13).

13. Appareil selon une ou plusieurs des revendications précédentes, **caractérisé en ce que** ledit moyen de refroidissement de chambre (13) comprend un ou plusieurs dispositifs thermoélectriques (14) disposés à l'extérieur dudit corps (2), chacun desdits dispositifs thermoélectriques (14) étant associé à un système de refroidissement (15) alimenté avec un fluide de refroidissement qui circule entre un connecteur d'entrée (16) et un connecteur de sortie (17).

14. Appareil selon une ou plusieurs des revendications précédentes, **caractérisé en ce qu'**il comprend au moins un capteur de température (18) apte à mesurer la température de ladite zone de peau à traiter et à transmettre les données audit système de commande (104) pour la comment dudit moyen de génération d'énergie vibratoire (10) et dudit moyen d'alimentation en fluide de traitement (102), ledit capteur de température (18) étant disposé de manière à être en face d'une fenêtre (6) prévue dans ladite paroi amovible (4) à l'intérieur de ladite chambre (3).
